# EUROPEAN PATENT APPLICATION

(11) **EP 1 167 530 A2**
(43) Date of publication of application: **02.01.2002**
(21) Application number: 01202225.7
(22) Date of filing: 11.06.2001
(51) Int. Cl.: C12N 15/82, C07K 19/00, C07K 14/08, A61K 39/21, A01H 5/00

(54) **PVX capsid-antigen fusion protein, chimaeric viral particles displaying it on the capsid, plants infected with said particles, their uses and compositions comprising them**

(30) Priority: 16.06.2000 IT RM000327
(71) Applicant: Ente per le Nuove Tecnologie, l'Energia e l'Ambiente - ENEA, 00196 Rome (IT); ISTITUTO SUPERIORE DI SANITA', 00161 Roma (IT)
(72) Inventor: Benvenuto, Eugenio, 00144 Roma (IT); Marusic, Carla, 00123 Roma (IT); Belardelli, Filippo, 00152 Roma (IT); Rizza, Paola, 00040 Monteporzio Catone RM (IT); Capone, Imerio, 00040 Monteporzio Catone RM (IT)
(74) Representative: Tonon, Gilberto

(57) **Abstract**

The present invention relates to a fusion protein comprising in the amino-terminal part an antigenic determinant having a length less than or equal to 35 amino acids and a percentage of hydrophilic amino acids such as to endow it with solubility in an aqueous environment, fused in frame with the PVX virus capsid protein.

The present invention further relates to chimaeric viral particles displaying the said fusion protein on the capsid, to plants infected with the said chimaeric viral particles, their uses, in particular for the preparation of pharmaceutical compositions suitable in the treatment and prevention of a pathology associated with pathogenic agents such as HIV-1, HCV, EBV and the influenza virus and compositions containing them, in particular vaccines.

## Description

### Field of the invention

The present invention relates to the field of recombinant plant viruses expressing, on their surface, antigenic regions of proteins from viral or bacterial pathogenic organisms, and to their use as immunogens.

### State of the art

Recombinant plant viruses having antigenic heterologous peptides on their surface are known in the art, as also is their use in vaccines (1,2).

This applies in particular to icosahedral (CPMV: cowpea mosaic virus; TBSV: Tomato Bushy Stunt Virus) or helicoidal (PVX: Potato virus X; TMV: Tobacco Mosaic Virus; AMV: Alfalfa Mosaic Virus) plant viruses engineered to display the antigenic peptide fused to the C- or N-terminal end of the CP (capsid protein), which is a site generally located in the external part of the said capsid, so as to give the peptide maximum exposure.

In view of their chemico-physical characteristics and their biological properties, these recombinant viral particles represent an advance over other known immunization systems. Thus, the recombinant plant viral vectors generally:
- do not involve the safety problems of traditional vaccines based on killed or live-attenuated pathogens, which are however characterized by a particular efficacy;
- tend to exhibit, at the same time, good immunogenicity and low cost, in contrast to the sub-unit vaccines, which are however characterized by a considerable safety in use;
- present an epitope which is not chemically modified, in contrast to vaccines wherein the antigenic protein is produced in heterologous systems by means of recombinant DNA technology;
- shall not necessarily be administered by parenteral route, in contrast to the traditional vaccines as well as sub-unit vaccines or those based on the antigenic determinant, as they can generally be administered both orally and intranasally (3,4).

Improving and development of recombinant plant viruses has highlighted problems regarding construction and functionality of the relevant chimaeric viral particles.

In particular, a first problem in this connection is that the presence of chimaeric proteins in the capsid can often cause difficulties in the recombinant viral particle assembly, which can be inhibited, as can also the long-distance movement of the assembled particle.

Both the length and the chemical-physical characteristics of the exogenous peptide (antigenic determinant) are factors contributing to the chimaeric viral particle formation. Self-assembly of the recombinant viral CP can in fact be prevented or made difficult both by the steric hindrance due to the dimensions of the fused polypeptide, and by the conformation of the exogenous peptide (antigenic determinant), due to its chemical nature and not to its dimensions.

In both cases, the interactions between the various molecules of recombinant CPs are rendered incompatible, with consequent inhibition of their assembly. Moreover, the presence of the exogenous peptide (antigenic determinant) can turn out into inhibition of the chimaeric viral particles transport from the site of primary infection to the rest of the plant, which is driven by the interactions between the virion and other proteins encoded by viral genes.

A second problem regarding construction and functionality of chimaeric viral particles is that the recombinant virus can show stability problems and in particular can recombine inside the plant cell. Recombination takes place by selection of mutants by deletion of sequences coding for exogenous peptide causing inhibition of virus assembly, which involves, in the majority of cases, loss of the exogenous peptide (antigenic determinant) together with some amino acid of the viral CP.

Moreover, even if a stable chimaeric viral particle not recombining inside the cell is obtained, problems may arise relating to the antigenicity and the immunogenicity of the epitope exposed on the chimaeric viral particles. In particular:
- exposure of the epitope on the capsid may be such as to maintain its antigenicity, but to confer little if any immunogenicity to the viral particle (accordingly administration of the epitope together with an adjuvant may be necessary);
- the recombinant viral particles, though endowed with immunogenicity, may not be capable of eliciting the production of neutralizing antibodies.

In particular, problems of assembly and stability have characterized development of the potato virus X (PVX) particles (5) displaying exposed on the capsid, fusion proteins comprising the CP (capsid protein) and a peptide of interest. In fact, vectors derived from the PVX virus are already known in the art and are widely used for expressing, in plants, heterologous proteins deriving from the expression in the viral vector of cloned genes expressed as accessory open reading frames, and not as parts of CP fusion proteins.

The only exception is the construction of a viral CP fused to the 2A peptide of the foot-and-mouth disease virus (FMDV). This peptide's function is to very efficiently catalyse the excision of the peptide bond of any protein or peptide fused to it. This makes it possible to express in plants chimaeric CPs and normal CPs including the chimaeric CPs in viral particle assembly. With this modified vector, CVPs carrying a reporter protein (GFP) (6) have been obtained. No example however has been reported regarding the possibility of using such a vector as a "system for presentation" of epitopes for vaccination purposes, nor simply that particles so obtained have immunogenicity.

In this connection it should be pointed out that following the adoption of this procedure, non-recombinant CPs are preferred over recombinant CPs in the viral particle assembly. This result in that the assembly of normal CPs is favoured, and in that following adoption of this technique viral particles having recombinant CPs in the capsid present them at a particularly low concentration.

It is not surprising accordingly that recombinant PVX virus up to now has not been used as an antigen presentation system.

In fact the impossibility of presenting a certain amount of antigenic determinants displayed on the capsid is an important obstacle to the use of the virus for vaccination purposes as it is well known in the art that for purposes of immunization it is necessary to have a presentation system concentrating on the surface the greatest number of antigenic determinants.

This is valid for any antigenic determinant, and even more so for antigenic determinants of pathogenic agents difficult to neutralize, as for instance HIV.

HIV is in fact known as one of the most difficult pathogen to counteract even by vaccine therapy, and an enormous number of difficulties so far has prevented the development of an effective prophylactic anti-HIV vaccine.

These difficulties derive first and foremost from the high degree of variability of the most strongly immunogenic regions of gp120 viral coating protein, and from the inaccessibility to the immune system of certain epitopes of gp41 protein that are crucial for the virus entry into the target cell, as well as from its retroviral nature.

Furthermore, for HIV in particular, the ability to obtain a response that is not only humoral but also cell-mediated is an essential condition for conferring immunity to the virus (7,8) even if effective humoral responses seem however to be important for the prevention of infection (9,10).

The production of virus neutralizing antibodies has proved to be particularly difficult. In this connection, not only are such antibodies generally present at very low levels in seropositive patients (11,12), but polyclonal serum preparations from these individuals also did not display an increased degree of protection in passive immunization tests in experimental animal models.

A number of perspectives have however opened up by the identification of an epitope of the gp41 coating protein of the HIV-1 virus that maps in the ectodomain C-terminal portion of the gp41 itself and that is highly conserved among the various subtypes of HIV (making it a candidate for the formulation of a multicomponent vaccine) (9). This epitope is recognized by a monoclonal antibody, denominated 2F5, that has been shown to be capable of neutralizing a broad spectrum of laboratory strains and primary isolates differing from HIV-1 (13), and of effectively protecting macaques from infection after exposure to infecting doses of SIV following passive transfer of the said antibody by the vaginal route, singly or combined with two other neutralizing monoclonal antibodies (14,15).

Despite these positive results, so far constructing an displaying system of the said epitope capable of eliciting an effective immune response, has not been possible.

On the basis of general knowledge concerning the ineffectiveness of methods of immunization based on peptides as such, methods of peptide exposure on surface antigens of animal viruses (16,17) and methods of conjugation to carrier proteins (18) have been used. In the first case, interesting results are obtained if the epitope is exposed at the immunogenic site of the haemagglutinin antigen of the influenza virus, demonstrating that outside of its natural context the epitope can induce neutralizing antibody responses. In the second case, however, it was demonstrated that if the peptide is conjugated to bovine serum albumin (BSA) in a single copy, the antibody responses that are obtained are considerably lower compared with the same carrier that contains multiple copies of the epitope, strengthening the hypothesis according to which the immune system is stimulated differently depending on the number of molecules of a particular epitope with which it interacts.

### Summary of the invention

The difficulties and problems described above have been overcome by the present invention, which relates to a fusion protein comprising:
- an amino-terminal portion comprising an antigenic determinant having a number of amino acid residues less than or equal to 35 and a percentage of hydrophilic amino acids such as to endow the said antigenic determinant with solubility in an aqueous environment, and
- a carboxy-terminal portion comprising a protein of the capsid of a PVX virus or a variant of the said capsid protein,
the said amino-terminal portion being fused to the said carboxy-terminal portion in such a way that the said antigenic determinant is in frame with the said capsid protein or with the said variant.

In fact, in presence of a polynucleotide having the functions of a PVX genome, and in particular in presence of recombinant PVX genomes also coding for the said fusion protein, the said fusion protein has shown self-assembly, giving rise to a viral particle that is correctly assembled and hence stable.

Correct assembly is demonstrated by the ability of the chimaeric virion to give rise to all the phases of the infective symptoms both in terms of spatial distribution and in terms of temporal kinetics, in a manner fully comparable to the wild-type virion.

In this connection, the chimaeric viral particles obtained have a stability similar to the wild type particles, as demonstrated by the fact that after repeated cycles of infection and production, the exogenous sequences are unchanged both at the genomic level and in the capsid, without rearrangements or deletions of the relevant nucleotide or -amino acid sequences.

For constructing the fusion proteins of the present invention it is possible to use antigenic determinants derived from pathogenic agents, such as bacteria or viruses, in particular from viruses of the following families: caliciviridae, togaviridae, flaviviridae, coronaviridae, arenaviridae, reoviridae, hepadnaviridae, parvoviridae, papovaviridae, adenoviridae, herpeviridae, poxviridae, picornaviridae, ortho and paramyxoviridae, rhabdoviridae, bunyaviridae, and retroviridae.

In particular the antigenic determinants of the fusion protein of the present invention are antigenic determinants of a pathogenic agent selected from the group consisting of HIV, HCV, EBV and influenza virus, more particularly the determinants derived from an antigen selected from the group consisting of gp120 of HIV, gp41 of HIV, EBNA-3A of EBV, EBNA-3B of EBV, EBNA-3C of EBV, matrix protein of the influenza virus, haemagglutinin of the influenza virus, NS3 of HCV, E2 of HCV and E1 of HCV, and specifically the antigenic determinants having a sequence reported in the annexed sequence listing as SEQ ID NO:1, SEQ ID NO:3, SEQ ID NO:5, SEQ ID NO:7, SEQ ID NO:9, SEQ ID NO:11, SEQ ID NO:13, SEQ ID NO:15, and from SEQ ID NO:17 to SEQ ID NO:30.

Object of the invention are also mimotopes of the said antigenic determinants, i.e. epitopes which, though having different primary structure compared to the epitopes to which they are referred, are characterized by a secondary and tertiary structure displaying antigenic and immunogenic properties entirely comparable to their relevant counterpart.

The chimaeric viral particles having on the capsid the fusion proteins of the present invention that include in the amino-terminal part the antigenic determinants enumerated above (though this consideration extends on the base of the state of the art to the relevant mimotopes in addition to the advantages enumerated above) are in fact surprisingly good immunogens, verified by the good result of ELISA tests carried out with specific antibodies and with sera taken from mice immunized by means of the recombinant viral particles of the present invention.

The fusion proteins containing the epitope of SEQ ID NO:1 (see below) in the amino-terminal portion are of particular interest. With regard to the carboxy-terminal portion of the fusion protein of the present invention, the capsid protein of the PVX X3 strain was used, but it can be constituted from the capsid protein (CP) of any strain of the PVX virus, as well as from a variant thereof. For the purposes of the present invention, variant of the capsid protein of PVX are any polypeptide, for example corresponding to or comprising a mutant or a part of the said protein, capable of ensuring, in the presence of a polynucleotide having the functions of the PVX genome in a suitable environment, assembly of the chimaeric viral particle. This includes in particular polypeptides that are part of the capsid protein, and indeed the polypeptide having the 4-292 amino acids of the said protein shall be considered a preferred embodiment.

The present invention also includes the variants of the fusion proteins of the present invention. Variant of the fusion protein of the present invention is any polypeptide that corresponds to or comprises a mutant of the said protein or part thereof, capable as such, in the presence of a polynucleotide having the functions of PVX genome, of giving rise to chimaeric viral particles that are able to assemble correctly and/or present immunogenicity.

The present invention also relates to all polynucleotides (consisting of natural or synthetic DNA, RNA or cDNA) coding for the fusion proteins of the present invention or for their variants. A case of particular interest is the one wherein the said polynucleotides coding for the fusion proteins of the present invention are included in polynucleotides having the functions of the PVX genome, or with the latter coexisting in the genome of a PVX virus to form recombinant PVX genomes or their functional equivalents, which are also to be regarded as an object of the present invention.

Object of the invention are also chimaeric viral particles having, on the capsid, at least one of the fusion proteins of the present invention or one of their variants. Particles that include, in the capsid, a recombinant genome also coding for the fusion protein of the present invention and/or for one of its variants are particularly preferred.

In addition to the typical advantages of the plant viral particles of the state of the art (in particular safety for mammals and humans), the said particles show considerable stability and capability of correct assembling. Above all, however, for the purposes of immunization they have the notable advantage of presenting to the host's immune system numerous molecules of immunogenic peptide concentrated on a single particle thus functioning as a carrier. The plant virion is then made up of about 1500 molecules of recombinant CPs that display just as many molecules of antigenic determinant-peptide, with obvious advantages over the state of the art.

The chimaeric particles of the present invention in fact all exhibit a surprising immunogenic capability, in addition to a notable stability.

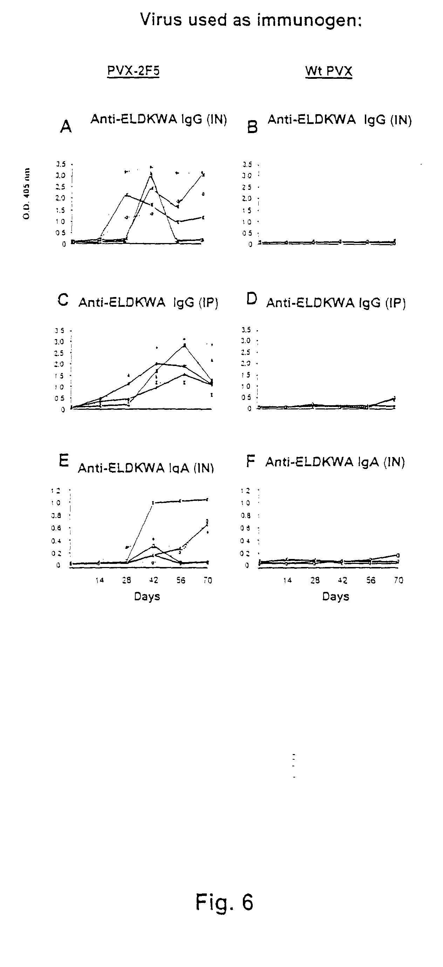

In particular, among all the chimaeric viral particles produced, the one containing the epitope recognized by the 2F5 monoclonal antibody (ELDKWAS) SEQ ID NO:1) as a result of immunization experiments in immunocompetent mice was found to be extremely stable and also capable of eliciting an excellent immune response, considering both specificity and antibody titres. In fact, only the sera of the mice immunized with the CVPs (chimaeric viral particles) exhibited reactivity to the ELDKWAS peptide, with antibody concentrations comparable to those reported in experimental protocols using traditional systems of epitope presentation (17, 18, 19), which includes the problems of safety and production costs of the art which limit their possible use for vaccine purposes. The results are illustrated extensively below and are shown schematically in Fig. 6.

Moreover, these particles, which constitute a preferred embodiment, have demonstrated capability of self-reproduction with a faster kinetics than the wild type ones (the time to appearance of the symptoms of infection is approx. 1/3 earlier) and the yields that are obtained in preparation of the CVPs are twice as high (being 0.4-0.5 mg/g of fresh tissue for PVX-2F5 and 0.2-0.3 mg/g of fresh tissue for PVX) compared with those of non-chimaeric particles, with identical conditions adopted both for infection and for purification.

In any case an advantage of the chimaeric particles of the present invention is in fact given by the possibility of administering these particles not only by the parenteral route but also by the mucosal, intranasal and/or oral route. Following administration of the particles of the present invention by these routes, both secretory and serum IgAs specific for the immunogen were found after intranasal immunization, which confirms the validity of the strategy for inducing immunity both at the mucosal and at the systemic level. Notably the result was achieved with relatively small amounts of immunogen (50 µg/dose/week) and without the use of an adjuvant. The results are greatly improved by using immunomodulator cytokines as adjuvants. In particular the type I interferon used as an adjuvant in vaccinal strategies using the chimaeric particles of the present invention produce a surprising potentiating effect of the immune response, both humoral and cell mediated. In this sense, these adjuvants, as well as any other adjuvant that can be identified by a person skilled in the art on the basis of his knowledge, can not only be supplied together with the particle in suitably prepared compositions, which must be regarded as included in the present invention, but can be produced in the same plant hosts in which the immunogenic chimaeric viral particle is produced. In this sense, the object of the present invention also includes a kit for the immunization of a mammal and, in particular, of human beings comprising
- a composition such as those described above that do not include an adjuvant; and
- a composition that includes an adjuvant together with a pharmaceutically compatible vehicle,
for simultaneous separate or sequential use in the immunization said mammal.

The antibody response obtained with the chimaeric viral particles of the present invention, to HIV, considered as activity capable of inhibiting HIV infection in *in vitro* neutralization assays, proved to be particularly surprising. The neutralization titres range from 1/40 to 1/120, corresponding to the limiting dilutions at which the sera display a 90% infection inhibition, according to the animals individual response.

Accordingly the chimaeric viral particles of the present invention displaying antigenic determinants of HIV on the capsid and in particular the antigenic determinant having SEQ ID NO:1, constitute particularly preferred embodiments.

A further advantage of the chimaeric viral particles of the present invention is that they are a particularly effective system for carrying the antigen, suitable both for parenteral administration and for intranasal or oral administration.

This constitutes a notable advantage with respect to use of the particle of the present invention for vaccinal purposes. Thus, administration by the parenteral route involves a reaction only at the systemic level and not at the mucosal level. In this connection, as it is well known that the majority of pathogens make use of the mucosal routes as routes of access to the host, vaccination strategies that include the induction of a mucosal immune response gives the advantage of permitting an immediate antibody response to the pathogenic agent.

Such a response can be elicited following administration of the antigen directly in the mucosae or by the intranasal or oral route, this latter route of administration being especially easy and advantageous.

Mucosal administration of antigens in the absence of adiuvants result in immunity or tolerance depending upon several factors, such as the type of antigen, the dose, and the cytokine milieu produced following antigen exposure. Interestingly, a strong immune response has been observed following repeated intranasal immunizations, suggesting that CVPs act as adjuvants, which are generally capable of breaking tolerance, shaping the response towards immunity. Typically, the breaking of tolerance is associated with a T helper-1 (Th-1) type of immune response. Interestingly, the antibody responses observed in mice, immunized intranasally with CVPs were predominantly of the IgG2a isotype, suggesting a Th1-type immune response. The polarization of the response towards this T helper cell type is considered a reliable correlate of immune protection in viral infections, thus the immune response induced by CVPs exhibited a Th profile potentially consistent with protection against viral infection.

According to the present invention the immunogen can also be administered without purification procedures directly by the oral route, carried by the tissue of the edible plant. In order to obtain an efficient immunization by the oral route, various obstacles must be overcome, such as immunogen degradation at acidic pH or by proteases in the gastrointestinal tract and the immunogen short exposure to the immune response sites.

In this connection, recent studies (2) demonstrate that plant systems can function as efficient means for vaccines production and administration. Moreover, like systems based on liposomes and microcapsules, plant cells are widely expected to be able to naturally protect and carry the antigen they include, permitting its release in the gastrointestinal tract and favouring its presentation to the lymphoid system associated with the latter. This aspect has important implications, especially for infective agents transmitted via mucosae and for which local immunity assumes a crucial importance. Furthermore, oral administration is generally easier and non-traumatic especially in subjects such as the elderly and children.

Further advantages of the present invention are that the viral particle thus produced constitutes a system for the antigen production characterized by very low costs, as it only requires the infection of plant hosts and growing them in controlled glasshouses, and that it guarantees a high level of safety of the product to be administered.

Plant hosts of the present invention can be monocotyledonous and dicotyledonous plants, or any tissue or part of them. In particular, the object of the present invention includes cells or protoplasts, derived from the plants of the present invention, which express the viral particles of the present invention, also constituted in cellular or tissue aggregates.

In view of the properties reported above, the present invention also includes the viral particles of the invention or the plants or plant cells comprising them for use as a medicament. In particular, the present invention relates to the use of the said particles for the preparation of medicaments suitable in the preventive treatment of infectious diseases, in particular AIDS, viral hepatitis, influenza and a pathology associated with the presence of the EBV virus.

The present invention also relates to compositions comprising at least one fusion protein as defined above or one of its variants, at least one viral particle as defined above or one of its variants, together with a compatible vehicle. A compatible vehicle for the purposes of the present invention is any vehicle among those known in the art, chemically and/or pharmaceutically compatible with the polypeptide and/or with the viral particle of the present invention.

Compositions including extracts, purified or not, of the chimaeric viral particles from the plants as defined above, are particularly important.

In particular, the invention relates to the compositions defined above consisting in vaccines comprising the viral particles of the present invention. All formulations involving purification of the chimaeric viral particles, the relevant preparation also in non-purified form and their administration, for the purpose of vaccination, by the classical routes and by the intranasal and oral route, are to be regarded as included.

Preparation and administration of the particles of the present invention can be carried out by any the techniques known in the art to be suitable to this purpose. In particular, it is possible to use the administration technique including stimulation of human dendritic cells (DCs).

In fact, PVX-2F5 exhibits a powerful immunogenic potential, which includes the generation of a neutralizing response, not only in mice but also in a human context. Human DCs pulsed with PVX-2F5 can trigger *in vitro* proliferation of autologous PBLs, suggesting that PVX-derived CVPs are able to activate immune cell responses. Therefore, we investigated whether plant-derived CVPs carrying ELDKWAS epitope could be used to stimulate human immune response against HIV-1. Severe Combined Immunodeficient (SCID) mice immunized with human autologous monocyte-derived DCs pulsed with PVX-2F5 showed significant levels of Igs specific for 2F5 epitope. Moreover, human antibodies showed neutralization activity against HIV.

The fusion proteins, the viral particles and the plants of the present invention can therefore be produced using methods known in the art. In general, it is possible to use recombinant DNA technology for constructing viral CPs fused to the peptides that constitute the fusion proteins of the present invention, by a first stage effected in E. coli and then by infection of the plant hosts with plasmids containing the recombinant genome of the virus, using classical methods of plant virology.

In particular, for the preparation of a fusion protein of the present invention it is possible to use a process comprising the following steps:
a. cloning the capsid protein as defined above, in a vector; and
b. cloning the antigenic determinant as defined above fused to the said capsid protein in the vector resulting from operation a.

The chimaeric viral particle of the present invention can be prepared by a process comprising the step of replacing capsid protein of a PVX virus, in the said PVX virus.

The plant of the present invention can be prepared by a process comprising the following steps:
a1. infecting a plant with a viral particle of the present invention;
b1. cultivating the plant obtained in outcome of step a1;
c1. extracting the said viral particle from the said plant.

The object of the present invention further includes the use of the fusion protein, the chimaeric viral particles, the plant and the plant cells of the present invention:
- for the preparation of drugs for the treatment of a pathology associated with a pathogenic agent of a bacterial or viral nature, in particular for the treatment of a pathology associated with a pathogenic agent selected from the group consisting of HIV, HCV, EBV and influenza virus; and
- for the derivation of diagnostic methods for a pathology associated with a pathogenic agent of a bacterial or viral nature, in particular for the derivation of diagnostic methods for a pathology associated with a pathogenic agent selected from the group comprising HIV, HCV, EBV and influenza virus.

The following are also included: the use of an antigenic determinant or of an antigen as defined above, or of a capsid protein of the PVX virus for the preparation of the fusion protein, of the chimaeric viral particle, of the plant or of the plant cells of the present invention; the use of the plant of the present invention, for the production of a chimaeric viral particle of the present invention.

The invention will be better described with the aid of the annexed figures.

### Description of the figures

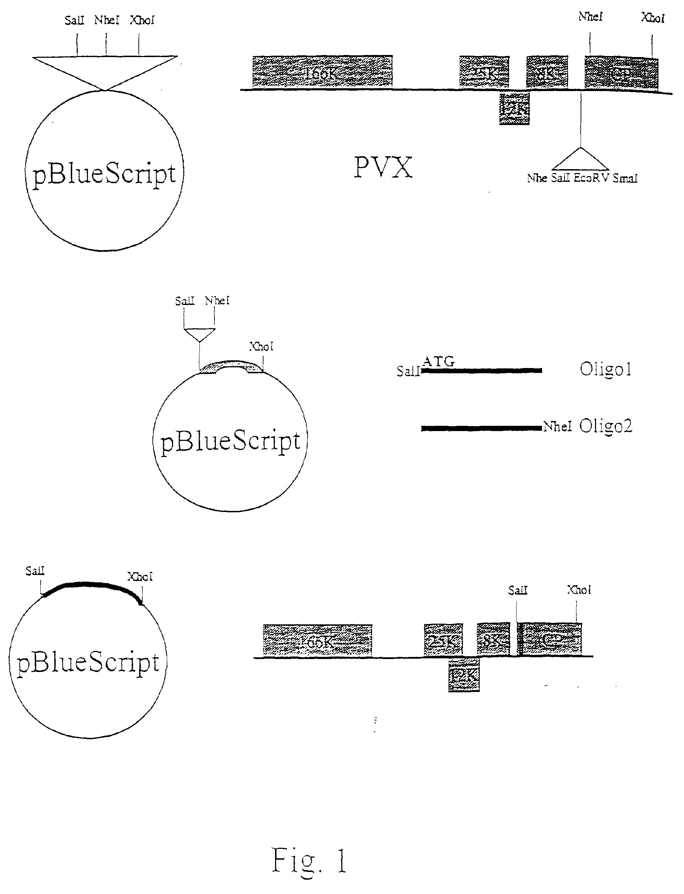

Figure 1 shows the scheme of the construction of recombinant PVX, for the production of CVPs that exhibit exogenous peptides on their surface. The wild-type CP of PVX was cloned as NheI-XhoI restriction fragment at the corresponding sites of pBlueScript. Double-helix oligonucleotides, coding for the exogenous peptides, were fused, in frame with the CP, using the SalI-NheI sites. The chimaeric CP thus obtained was inserted in the genome of PVX as a SalI-XhoI restriction fragment.

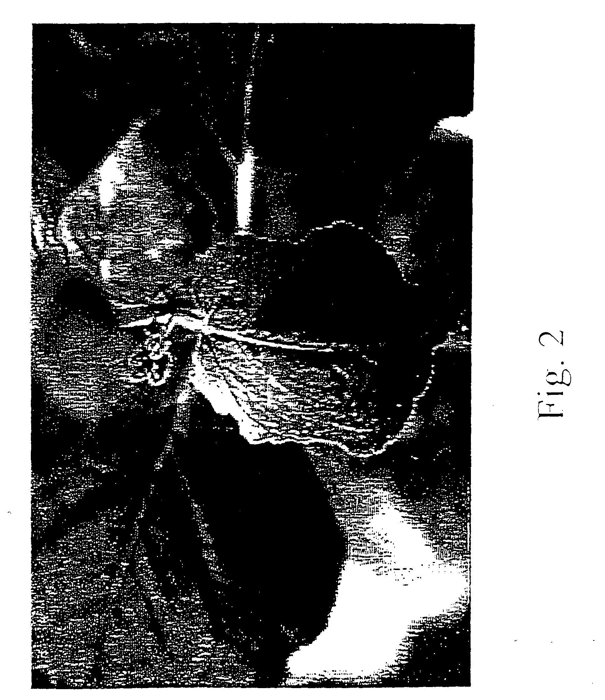

Figure 2 shows the leaf of a *Nicotiana benthamiana* plant infected with PVX -2F5 and with PVX Wt (wild-type) as control. The time of appearance and the symptoms observed for the plants infected with the engineered PVX were entirely comparable to those obtained with the PVX Wt (wild-type). Systemic leaf extracts were analysed by RT-PCR and ELISA for the purpose of verifying correct expression of the cloned sequence.

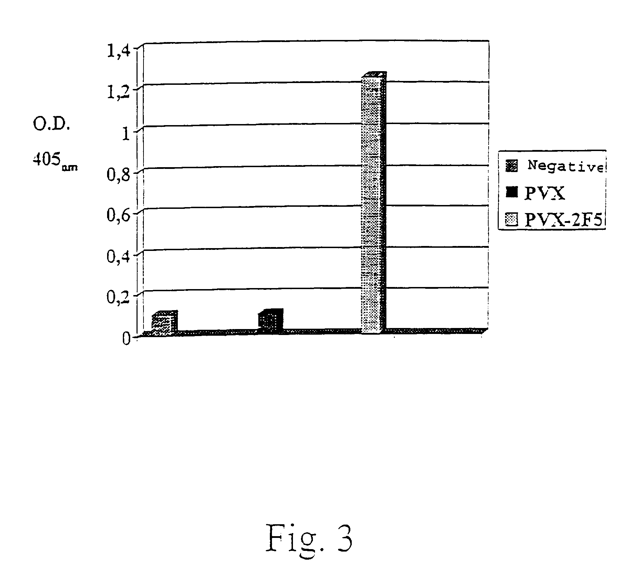

Figure 3 shows a diagram wherein the results of an ELISA assay carried out on the chimaeric viral particles extracted from plants infected with PVX and PVX-2F5, using the 2F5 monoclonal antibody, are reported according to the symbols shown in the figure, wherein on the ordinate the values of absorbance (O.D. 405 nm) are reported.

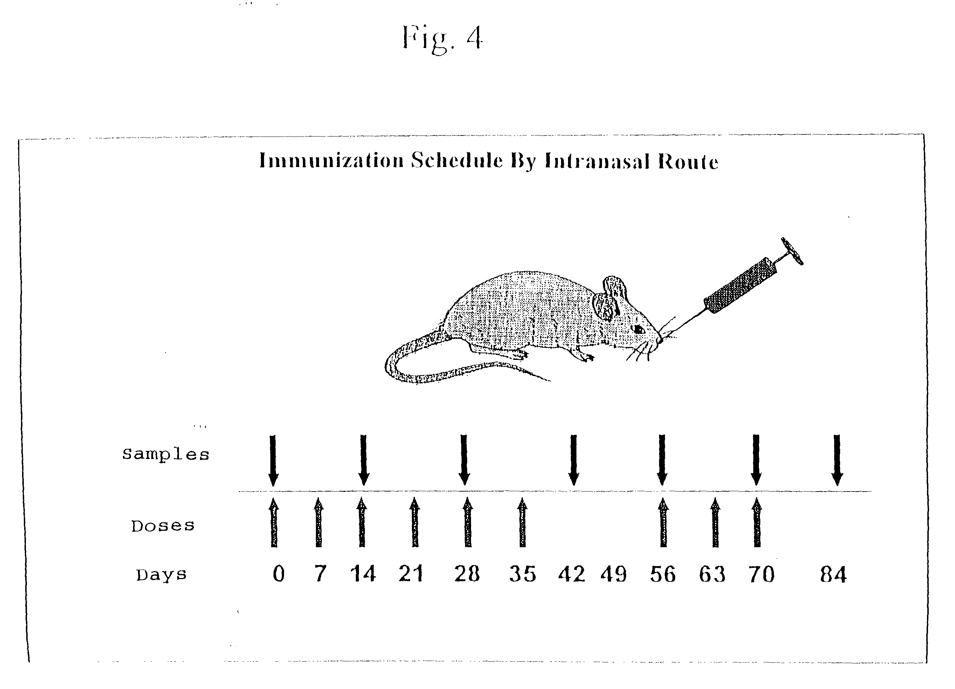

Figure 4 shows the immunization schedule by the intranasal route. The mice were immunized with 50 µg of virus per dose. The downward-pointing arrows indicate sampling, the upward-pointing arrows indicate the doses of virus administered, and the numbers represent days since the first dose.

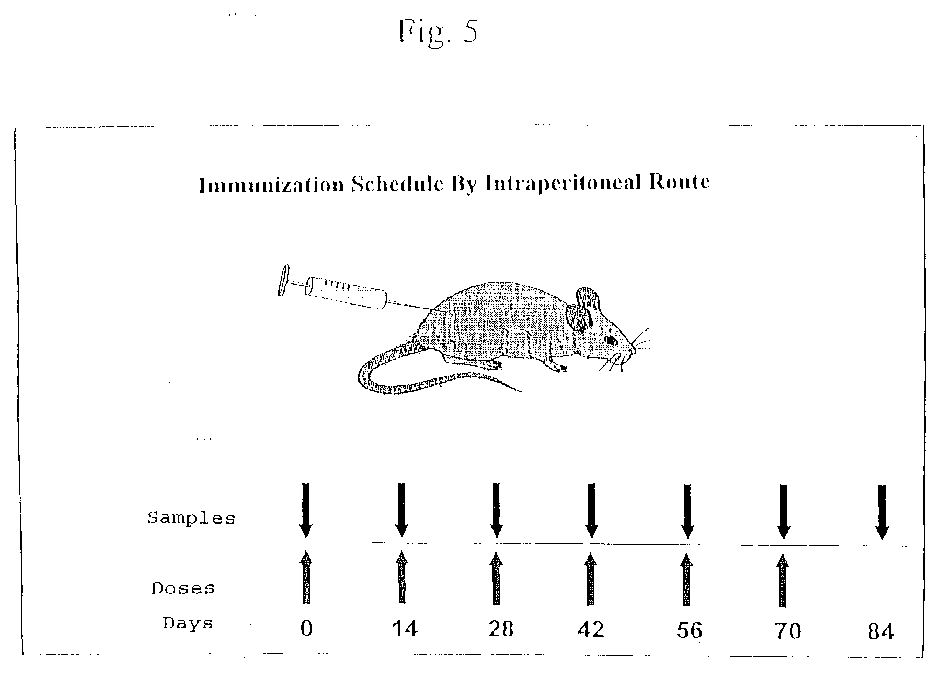

Figure 5 shows the immunization schedule by the intraperitoneal route. The mice were immunized with 50 µg of virus per dose. The downward-pointing arrows indicate sampling, the upward-pointing arrows indicate the doses of virus administered, and the numbers represent days since the first dose.

Figure 6 shows diagrams labeled from A to F reporting the results of ELISA tests carried out with sera diluted 1:50, using as immunogen PVX-2F5 (diagrams A, C and E) or wild type PVX (WtPVX) (diagrams B, D, and F). On each diagram days of the samplings are indicated on the abscissa, and the value of absorbance (O.D. 405 nm) on the ordinate. Each line represents the response of a single animal.

Diagrams A and B refer to IgG immune response detected after intranasal administration of respectively PVX-2F5 and wild type PVX using antibodies conjugated to the peroxidases directed against IgG mouse antibodies.

Diagrams C and D refer to IgG immune response detected after intraperitoneal administration of respectively PVX-2F5 and wild type PVX using antibodies conjugated to the peroxidases directed against IgG mouse antibodies.

Diagrams E and F refer to IgA immune response detected after intranasal injection of respectively PVX-2F5 and wild type PVX using antibodies conjugated to the peroxidases directed against IgA mouse antibodies.

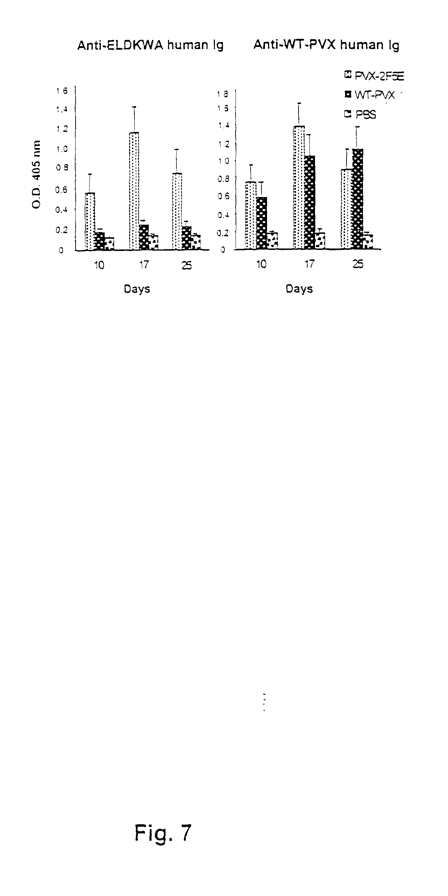

Figure 7 shows two diagrams reporting data on the detection of respectively anti-ELDKWAS and anti-Wild Type-PVX human immunoglobulins in sera of hu-PBL-SCID mice. Days of the samplings are indicated on the abscissa, and the value of absorbance (O.D. 405 nm) on the ordinate. Hu-PBL-SCID mice were intraperitoneally injected on days 3 and 10 with PVX-2F5, Wild Type-PVX-pulsed autologous DCs or with PBS. Histograms represent the mean optical density (405 nm) of four samples ± SD. Data obtained following PVX-2F5E, WT-PVX, and PBS administration are reported with different symbols as shown in the figure.

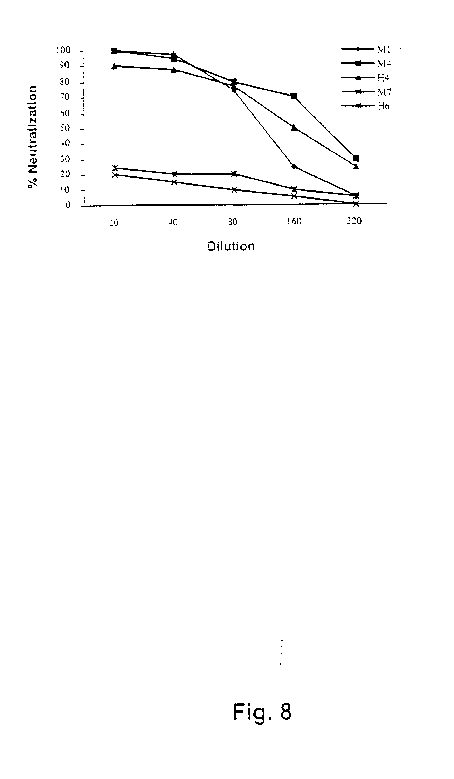

Figure 8 shows a diagram reporting data on the in *vitro* neutralization assay of HIV-1.
Neutralizing activity of sera collected at day 42 from mice immunized intranasally with PVX-2F5 (M1, M4) or Wild Type-PVX (M7), and at day 17 from Hu-PBL-SCID mice immunized with PVX-2F5-pulsed (H4) or Wild Type -PVX-pulsed (H6). DCs, have been determined by syncytium inhibition assay. In the graph percentage of neutralization (ordinate) are plotted against sera dilution (abscissa). Each line represents the neutralizing activity of a representative serum from one mouse in each group, according to the symbols shows in the figure.

### Detailed description of the invention

The present invention is based on the observation that polypeptides comprising the CP of PVX and, fused to its amino-terminal part, certain epitopes of the gp41 protein of the HIV virus with particular reference to the ELDKWAS epitope recognized by the 2F5 monoclonal antibody, give rise to correctly assembled chimaeric viral particles, which do not recombine following infection in a plant, and which are able to elicit a neutralizing antibody response with respect to the epitope displayed on the outside of the said viral particle.

In this connection, a series of fusion polypeptides was constructed using recombinant DNA technology techniques.

The experimental strategy adopted in order to achieve the objective was characterized by the following phases:

### 1. Preparation of the recombinant viral CPs

The pBlueScript (pBS) plasmid was used for this purpose, suitably modified in its polylinker so as to include certain sites for restriction enzymes, necessary for the genic fusions between exogenous peptides and viral CP.

The exogenous peptides used are those derived from the HIV-1 coating protein and from antigenic proteins of other pathogenic agents such as HCV, EBV and the influenza virus, capable of being recognized by the host's immune system and of inducing antibodies that are able to interfere with the viral infection process. The peptides were selected on the basis of their immunogenic features.

In particular, the following were used:
a) the epitope recognized by the human monoclonal antibody 2F5 and having the canonical sequence ELDKWAS, or the said epitope comprising sequence variations in the first and/or in the last two amino acids (E, A and S), with the central nucleus corresponding to the LDKW sequence remaining unchanged (SEQ ID NO:1) ;
b) epitopes from the N and C terminal regions of the ectodomain of the gp41 of HIV-1 (including the peptides of sequence SEQ ID NO:3, SEQ ID NO:5, SEQ ID NO:7 and SEQ ID NO:9) ;
c) mimotopes of gp120 and gp41 (SEQ ID NO:11, SEQ ID NO:13 and SEQ ID NO:15), or alternatively epitopes which, though having different primary structure compared to the epitopes which they are referred to, are characterized by a secondary and tertiary structure such that they display antigenic and immunogenic properties entirely comparable to their relevant counterpart. On the basis of these characteristics, the sera of "long-term non-progressors" patients, i.e. subjects who, following seroconversion, do not show any progression of the disease in the long term, were used for identifying a series of mimotopes of gp120 and gp41 (19) of HIV-1 which were fused to the CP of PVX;
d) epitopes derived from antigens of other pathogenic agents including EBV (Epstein-Barr Virus) (including peptides of sequence from SEQ ID NO:17 to SEQ ID NO:24), influenza virus (including the peptides of sequence SEQ ID NO:25 and SEQ ID NO:26) and HCV (including the peptides of sequence from SEQ ID NO:27 to SFQ ID NO:30).

The viral CP used was the PVX capsid protein (in particular the one having the sequence SEQ ID NO:31), i.e. a protein comprising the amino acids 4-237, which maintains all the structural and functional features of the natural one, i.e. capable of producing stable viral particles, even in presence of exogenous peptides.

The nucleotide sequences coding for these peptides and for the CP were used for cloning. In particular, with regard to epitopes of HIV and CP, in particular polynucleotides were used that have the sequences reported in the annexed sequence listing as SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:32.

Once obtained, the gene fusions were sequenced in order to verify their correctness.

### 2. Infection of plant hosts with the recombinant virus

In order to produce chimaeric viral particles, the viral infective genome was obtained by replacing the viral CP wild type with the recombinant CP obtained in *vitro.* The recombinant viral genome, cloned in a plasmid allowing its replication in *E. coli,* was prepared from bacterial cultures and used for infecting suitable hosts (mainly tobacco, but also potato and tomato). On appearance of the infectious symptoms, leaf samples were taken and stored for subsequent analyses.

### 3. Verification of chimaeric viral particles stability

The presence of the exogenous peptide (antigenic determinant) and its stability within the viral genome were assayed using polymerase chain reaction (PCR) on total RNA extracted from leaves exhibiting symptoms of infection both at the level of the inoculated leaf, and at the systemic level.

These fusion polypeptides, once expressed in the plants, have shown the capability of correct self-assembling together with the recombinant genome of PVX, giving rise to a chimaeric viral particle. For production of a viral particle, the infected plant cell, utilizing a strong viral promoter derived from the cauliflower mosaic virus (CaMV), produces an mRNA corresponding to the genomic RNA of PVX and coding for the viral CP fused to the exogenous peptides, together with a specific viral RNA polymerase and with proteins involved in movement of the viral particle within the plant's tissues (5).

The results confirmed gene fusions integrity after 3 subsequent cycles of generation and production of chimaeric viral particles.

Following verification of the infection both at the level of the leaf and at the systemic level, the data obtained were indicative of the stability of chimaeric particles in which the capsid protein is a fusion protein having in the amino-terminal portion, an antigenic determinant with a length of less than 30-35 amino acids and hydrophilic amino acids, in sufficient amount to guarantee the stability of the said determinant in an aqueous environment. In particular, all the proteins tested gave a positive result.

### 4. Verification of the immunogenicity of chimaeric viral particles of the invention

The chimaeric viral particles of the invention were checked for immunogenic properties. For this purpose, the viral particles were extracted and submitted to a series of ELISA tests. In particular, with regard to particles wherein the fusion protein comprising the antigenic determinant having the sequence SEQ ID NO:1 is displayed, its presence and the correct capsid antigenicity of the corresponding chimaeric viral particles were demonstrated in each cycle with an ELISA test according to the results shown in Fig. 3.

The other proteins tested also gave good results in the ELISA test, demonstrating that as well as being characterized by stability, the fusion proteins tested show good immunogenicity.

### 5. Administration of the chimaeric viral particles to immunocompetent mice and analysis of the immune response

The purified chimaeric viral particles were used for immunizing immunocompetent mice via the intraperitoneal, intranasal and oral routes. To the latter were added groups of experimental mice treated with murine IFN-α as immunostimulating cytokine, which was also administered orally at various concentrations. The results demonstrated the validity of the exogenous peptide (antigenic determinant) presentation system, with an high titre antibody responses (the sera show reactivity to the peptide down to dilutions of 1:10000), good neutralizing capacity (up to 100%) and, above all, with mucosal induction of immunity (presence of secretory IgAs).

### 6. Administration of the chimaeric viral particles to the human-Peripheral Blood Lymphocytes (hu-PBL) in the Severe Combined Immunodeficient (SCID) mouse model.

Hu-PBL-SCID mice were immunized with human autologous monocyte-derived DCs (20) pulsed with PVX-2F5 or Wild Type-PVX and mice sera were analyzed for the presence of antigen-specific human antibodies at different time points. Sera from chimeric mice immunized with PVX-2F5-pulsed DCs showed significant levels of Igs specific for ELDKWAS peptide, while no anti-ELDKWAS epitope reactivity was detected in the sera of mice injected with wild type -PVX-pulsed DCs or in PBS-injected controls (Fig. 7). Both DC-injected groups raised a human antibody response to WT-PVX (Fig. 7).

### 7.Verification of neutralizing activity of hu-PBL-SCID and immunocompetent mice sera.

The HIV-1 neutralizing activity of sera obtained from both normal and hu-PBL-SCID mice was evaluated by a syncytium inhibition assay (21). Sera from mice intranasally immunized with PVX-2F5 showed a consistent capability to inhibit syncytia formation, as compared to controls (Wild Type -PVX). Sera from hu-PBL-SCID mice immunized with PVX-2F5-pulsed DCs also showed neutralizing activity. In fig. 8 data are reported on the neutralizing activity of both mouse and human representative individual serum.

### 8. Polypeptide administration to animals for vaccinal purposes

Administration was carried out according to a process comprising the following phases:
- infection of plant hosts with the genome of a recombinant plant virus (potato virus X or PVX) for producing the viral capsid fusion proteins (CP), as above;
- cultivation of the infected plants in suitable conditions to produce the recombinant CPs which, following self-assembly, produce chimaeric viral particles (CVPs) displaying the exogenous peptides on their surface;
- administration of the said CVPs to animals for vaccinal tests, not excluding simple production of antibodies against the peptide of interest.

Such an administration comprised administration of purified CVPs, raw extracts of plant material containing the CVPs, and unprocessed plant material. It was carried out on mice but could have been carried out on other mammals.

The results are reported on figs. 6-8.

Up to now a general description of the present invention has been given. With the aid of the following examples, a more detailed description of specific embodiments will be provided, with the aim of giving a better understanding of the objectives, features, advantages and operating conditions of the invention.

### EXAMPLES

### Example 1: Construction of vectors comprising a fusion protein with the epitopes of HIV

Construction of the fusion protein of recombinant PVX for the epitopes of HIV was obtained according to the strategy schematically shown in Fig. 1. The polylinker of the pBlueScript (pBS) plasmid was modified to insert SalI, NheI and XhoI restriction sites in order: the NheI site allow the insertion of any double-helix oligonucleotide coding for a peptide of interest in frame with the CP, whereas the other sites made it possible to replace the wild-type CP in the PVX genome with the chimaeric CP obtained in pBS. This strategy involved, in the specific case, deletion of the first 9 nucleotides (3 amino acids) of the PVX CP gene, including the translation starting trinucleotide (ATG). After annealing two single-helix oligonucleotides, a double-helix oligonucleotide coding for the epitope was obtained. The epitope is preceded by the translation start codon ATG and the coding sequence is flanked by the sticky ends of the SalI and NheI cloning sites; next the double-helix oligonucleotide was inserted in the corresponding pBs restriction sites wherein the gene of the PVX capsid protein, with the first 3 amino acids deleted, had previously been cloned, with the NheI and XhoI enzymes. In this way, a gene coding for a recombinant capsid protein carrying, on its N-terminus, as fusion peptide the epitope of interest, was obtained. This strategy was adopted for inserting the epitopes previously described.

The gene coding for the recombinant capsid protein was rescued as a SalI-XhoI restriction fragment and cloned in the same restriction sites of the PVX201 plasmid (10), containing the cDNA corresponding to the PVX genome, replacing the wild-type capsid protein with the chimaeric capsid protein.

### Example 2: Infection of the plants and production of the chimaeric viral particles (CVPs)

PVX201 plasmid was prepared from a culture of E. *coli* and used for infecting plants of *N.* benthamiana and *S. tuberosum.* Plasmidic DNA was then dispersed on two leaves of the plant to be infected (approx. 15 µg/leaf), after first treating the leaf with abrasives to allow penetration. After about 7 days, first symptoms of infection appeared at the level of the inoculated leaf, which in the further 4-5 days was transformed into systemic infection, detectable by the symptoms in the newly formed leaves at the top of the plant, shown in Fig. 2. During infection, the plants were kept in phytotrons at a temperature of 25°C and in circadian conditions of 14 h of light and 10 h of darkness. On appearance of the first symptoms at the systemic level, a small sample of leaf tissue was taken, and was submitted tc RT-PCR analysis to verify the presence of the exogenous sequence in the gene of the chimaeric fusion protein. The RT-PCR reactions were carried out using a Perkin-Elmer kit, in accordance with the manufacturer's instructions, using as template 1 µg of total RNA extracted from infected leaves (infected with the recombinant virus or with the wild-type virus) and using as primers, oligonucleotides corresponding to the exogenous peptide (antigenic determinant) (sense primer) and to the viral CP (antisense primer).

About 15 days after infection, leaves showing evident symptoms of infection were harvested and subjected to chimaeric viral particles extraction. The plant tissue was powdered in a mortar in presence of liquid nitrogen and the powder was then resuspended in the extracting buffer containing 0.1 M Tris-borate pH 6.8, and the viral particles were purified on a CsCl gradient. Then, an ELISA test was carried out to verify correct antigenicity of the epitope expressed on the chimaeric viral particles. Fig. 3 shows the ELISA test carried out on the chimaeric viral particle containing the ELDKWAS epitope (SEQ ID NO:1) and recognized by the 2F5 monoclonal antibody. The test was carried out by adsorbing, on an ELISA plate, 100 µl of an anti-PVX polyclonal antiserum, diluted 1:5000 in carbonate buffer, at 4° for approx. 16 h. After washing in 1x PBS, 50 µl of a foliar extract containing the chimaeric viral particles and 50 µl of 1x PBS were added, and the plates were incubated for 1 h at 37°C. Then, the plates were washed 3 times with 1x PBS, the 2F5 antibody, diluted 1:150, was added, and incubation was continued for 1 h at 37°C. After 3 further washings in 1x PBS, a human anti-IgG secondary antibody conjugated with peroxidase, diluted 1:5000, was added, and the staining reaction was started.

Plate was read at various time, with a maximum time of 30 min. Finally; the chimaeric viral particles were used for subsequent cycles of infection to evaluate the particles stability and check that no recombinations in the site of insertion of the sequence coding for the exogenous peptide (antigenic determinant) occurred. The ELISA test was repeated in each cycle.

### Example 3: Immunization of immunocompetent mice

The purified chimaeric viral particles were then used for immunizing immunocompetent mice in accordance with the schedules reported in Figs. 4 and 5, by the intraperitoneal and intranasal routes of administration.

### Example 4: Analysis of the immune response

Sera obtained from the immunized mice following the experiments reported in Example 3 were aliquoted and stored at -20°C for the entire duration of the experiment. At the end of the experiment, ELISA assays were carried out to evaluate the presence of various classes of immunoglobulins, in particular serum IgG and IgA and faecal IgAs specific to the ELDKWAS epitope, using a synthetic peptide containing the amino acid sequence. The peptide was diluted to a concentration of 4 µg/ml in carbonate buffer and 100 µl of this solution was distributed into each well of a titration plate, which was incubated at 4°C for 16 hours. Next, 3 washings were carried out in 1x PBS/0.2% Tween 20 and 1 washing in 1x PBS. Then a solution containing 2% milk (formulat 1, Dicofarm)/0.2% gelatin was added to each well and the plate was incubated at 37°C for 2 hours. After 3 washings in 1x PBS/0.2% Tween 20 and 1 washing in 1x PBS, the sera diluted 1:50 in 2% milk were added and incubation was continued at 4°C for 16 hours. Then the plate was washed as described above and the developer antibody conjugated with the peroxidase enzyme was added. After incubation at 37°C for 1 hour, the enzyme substrate was added and the reaction was quantified using spectrophotometry at 405 nm. The data presented in Fig. 6 demonstrate that the sera of the mice have an excellent specific response to the peptide of interest with production both of serum IgG and IgA and of faecal IgAs, indicating that an immune response had occurred at the mucosal level, which was moreover evident in the intranasal immunization protocol.

### Example 5: Hu-PBL-SCID mouse model

*Dendritic cells preparation.* Peripheral blood mononuclear cells were obtained from the blood of healthy donors by standard Ficoll-Paque density gradient centrifugation (Seromed). Monocytes were then isolated by subsequent Percoll density gradient centrifugation (20) and cultured in Lipopolysaccharide (LPS)-free flasks (Costar) at the concentration of 2x10⁶ cells/ml in RPMI 1640 (GIBCO BRL) supplemented with 10% FCS, 500 U/ml GM-CSF (R&D System) and 500 U/ml type I consensus IFN (specific activity 10⁹ U/mg protein; Yamanouchi), at 37 °C in 5% CO₂. After 3 days, non-adherent cells were collected and characterized for DC differentiation markers by FACS (Fluorescence Activate Cell Sorter) analysis as previously described (20). For pulsing, DCs (10⁷) were incubated with 500 µg of PVX-2F5 or Wild Type-PVX (100 µg/ml) for 2 hrs at 37 °C. After extensive washing with culture medium, DCs were used to immunize SCID mice previously reconstituted with autologous monocyte-depleted PBLs (2x10⁶ cells/mouse). Four week old CB17 scid/scid female mice (Charles River), housed under specific pathogen-free conditions, were intraperitoneally injected with 30x10⁶ monocyte-depleted hu-PBLs, obtained from the blood of healthy donors, resuspended in 0.5 ml RPMI 1640. Three and ten days after reconstitution, mice were intraperitoneally injected with 2x10⁶ human autologous DCs, previously pulsed with PVX-2F5 or with Wild Type -PVX (DCs injected ten days after mice reconstitution were obtained from autologous peripheral blood mononuclear cells frozen at the time of preparation). On days 10, 17 and 25, blood samples were collected and anti-ELDKWAS antibody titers in the sera were checked by ELISA. *Neutralization assay.* Neutralizing activity was tested by measuring the ability of sera of inhibiting virus-induced syncytia formation in a standardized virus cell system. The methods is adapted from P. Nara et al. (21). In such a test, a single infectious unit of virus infects a single cell and leading to a distinct response, that is the formation of a multinucleated giant cell. The addition of antibody with neutralizing activity can prevent this event and a quantitative measure of the residual infectivity can be determined. In the assay, the C8166 target cells (an HTLV-1 transformed human umbilical cord CD4+ lymphoblastoid cell line), and the T-tropic HIV-1 IIIB virus prototype were used. Mouse sera, inactivated at 56°C for 30', were diluted twofold in medium and distributed in 96 well-microtiter plates, four replicas (50 µl) per each dilution. An equal volume of virus (100 TCID₅₀, Tissue Culture Infective Dose) was then added and virus-serum mixture was incubated for 2 hours at room temperature. 5x10⁴ cells per well were then added in a final volume of 200 µl, and the plate further incubated at 37°C in 5% CO₂. After five days, the number of syncytia was counted in each well under a microscope and the mean percentage of neutralization estimated by the formula 1- Vₙ/V₀, where Vₙ is the mean number of syncytia in antibody treated cultures and V₀ is the mean number of syncytia in virus control cultures (21).

Up to now the present invention has been described referring to embodiments presented as non-limiting examples. A person skilled in the art can identify further embodiments included in the scope of the annexed claims.

### References

1. Arntzen CJ: High-tech herbal medicine: plant-based vaccines. Nature Biotechnol. 1998, 15:221-222.
2. Arntzen CJ: Pharmaceutical foodstuffs - Oral immunization with transgenic plants. Nature Med. 1998, Vaccine supplement 4:502-503.
3. Brennan FR et al.: Chimeric plant virus particles administered nasally or orally induce systemic and mucosal immune responses in mice. J. Virol. 1999, 73:930-938.
4. Modelska A et al.: Immunization against rabies with plant-derived antigen. Proc. Natl. Acad. Sci. USA 1998, 95:2481-2485.
5. Baulcombe DC et al.: Jellyfish green fluorescent protein as a reporter for virus infections. Plant J. 1995, 7:1045-53.
6. Lacomme C et al.: Genetic engineering and the expression of foreign peptides or proteins with plant virus-based vectors. In "Genetic Engineering", Setlow JK ed., Plenum Press, New York, 1998,20:225-237.
7. Burton DR and Moore JP: Why do we not have an HIV vaccine and how can we make one? Nature Med. 1998, Vaccine supplement 4:495-498.
8. Heneey JL et al.: Immune correlates of protection from HIV and AIDS-more answers but yet more questions. Immunol. Today 1999, 20:247-251.
9. Burton DR. A vaccine for HIV type 1: the antibody perspective. Proc. Natl. Acad. Sci. U S A 1997, 94:10018-10023.
10. Poignard P et al.: Neutralizing antibodies have limited effects on the control of established HIV-1 infection in vivo. Immunity 1999, 10:431-438.
11. Moore JP et al.: Inter and intraclade neutralization of human immunodeficiency virus type 1: genetic clades do not correspond to neutralization serotypes but partially correspond to gp120 antigenic serotypes. J. Virol. 1996, 70:427-444.
12. Moog C et al.: Autologous and heterologous neutralization antibody responses following initial seroconversion in human immunodeficiency virus type 1-infected individuals. J. Virol. 1997, 71:3734-3741.
13. Muster T. et al.: A conserved Neutralizing epitope on gp41 of Human Immunodeficiency Virus Type 1. J. Virol. 1993, 67: 6642-6647.
14. Baba TW et al.: Human neutralizing monoclonal antibodies of the IgG1 subtype protect against vaginal mucosal simian/human immunodeficiency virus infection. Nature Med 2000, 6:200-206.
15. Mascola JR et al.: Protection of macaques against vaginal trasmission of a pathogenic HIV-1/SIV chimeric virus by passive infusion of neutralizing antibodies. Nature Med. 2000, 6:207-210.
16. Muster T. et al.: Cross-neutralizing activity against divergent human immunodeficiency virus type 1 isolates induced by gp41 sequence ELDKWAS. J. Virol. 1994, 68:4031-4034.
17. Eckart L. J.et al.: Immunogenic presentation of a conserved gp41 epitope of human immunodeficiency virus type 1 on recombinant surface antigen of hepatitis B virus. J. Gen. Virol. 1996, 77:2001-2008.
18. Lu Y. et al.: Epitope-vaccine induces high levels of ELDKWA-epitope-specific neutralizing antibody. Immunol Invest. 2000, 29:41-50.
19.Scala G. et al.: Selection of HIV-specific immunogenic epitopes by screening random peptide libraries with HIV-1-positive sera. J. Immunol. 1999, 162:6155-6161.
20. Santini, S.M., Lapenta ,C., Logozzi, M., Parlato, S., Spada, M., Di Pucchio, T. and F. Belardelli. 2000. Type 1 interferon as a powerful adjuvant for monocyte-derived dendritic cell development and activity in *vitro* and in Hu-PBL-SCID mice. J. Exp. Med. 191: 1777-1788.
21. Nara, P.L., Hatch, W.C., Dunlop, N.M., Robey, W.G., Arthur, L.O., Gonda, M.A. and P.J. Fischinger. 1987. Simple, rapid, quantitative syncytium-forming microassay for the detection of human immunodeficiency virus neutralizing antibody. AIDS Res. Hum. Retroviruses 3: 283-302.

## Claims

1. A fusion protein comprising:
- an amino-terminal portion comprising an antigenic determinant having a number of amino acid residues less than or equal to 35 and a percentage of hydrophilic amino acids endowing said antigenic determinant with solubility in an aqueous environment, and
- a carboxy-terminal portion comprising a capsid protein of a PVX virus or a variant of said capsid protein,
said amino-terminal portion being fused to said carboxy-terminal portion in such a way that said antigenic determinant is in frame with said capsid protein or with said variant.

2. The fusion protein according to claim 1, wherein said antigenic determinant is an antigenic determinant of a pathogenic agent selected from the group consisting of HIV, HCV, EBV and influenza virus.

3. The fusion protein according to claim 2, wherein said antigenic determinant is derived from an antigen selected from the group consisting of gp120 of HIV, gp41 of HIV, EBNA-3A of EBV, EBNA-3B of EBV, EBNA-3C of EBV, matrix protein of the influenza virus, haemagglutinin of the influenza virus, NS3 of HCV, E2 of HCV and E1 of HCV.

4. The fusion protein according to claim 3, wherein said antigenic determinant is selected from the group consisting of the antigenic determinants having a sequence reported in the annexed sequence listing from SEQ ID NO:1, SEQ ID NO:3, SEQ ID NO:5, SEQ ID NO:7, SEQ ID NO:9, SEQ ID NO:11, SEQ ID NO:13, SEQ ID NO:15, and from SEQ ID NO:17 to SEQ ID NO:30.

5. The fusion protein according to claim 3, wherein said antigenic determinant is an antigenic determinant having the sequence reported in the annexed sequence listing as SEQ ID NO:1.

6. The fusion protein according to any of claims 1 to 5, wherein said capsid protein of a PVX virus has the sequence reported in the annexed sequence listing as SEQ ID NO:31.

7. A polynucleotide coding for the fusion protein according to any claims 1 to 6 and/or for a variant of said fusion protein.

8. A polynucleotide having the functions of a PVX virus genome comprising the polynucleotide according to claim 7.

9. The polynucleotide according to claim 8, wherein said polynucleotide having the functions of a PVX virus genome is the genome of a PVX virus.

10. A chimaeric viral particle **characterized in that** it contains as capsid protein at least one fusion protein according to any of claims 1 to 6, said chimaeric viral particle having, displayed on the outside, the antigenic determinant of the amino-terminal portion of said fusion protein.

11. The chimaeric viral particle according to claim 10, wherein the viral genome is the polynucleotide according to claim 8 or 9.

12. A plant **characterized in that** it comprises at least one chimaeric viral particle according to claim 10 or 11.

13. Plant cells **characterized in that** they are derived from the plant according to claim 12 and **in that** they express the chimaeric viral particle according to claim 10 or 11.

14. The plant cells according to claim 13, said cells being constituted in cellular or tissue aggregates.

15. A pharmaceutical composition comprising a fusion protein according to any of claims 1 to 6, a chimaeric viral particle according to claim 10 or 11, an extract from the plant according to claim 12 and/or plant cells according to claim 13 or 14, together with a chemically or pharmaceutically compatible vehicle.

16. The pharmaceutical composition according to claim 15, wherein said composition further comprises an adjuvant.

17. The pharmaceutical composition according to claim 15 or 16, wherein said composition is formulated for oral administration.

18. The pharmaceutical composition according to claim 15 or 16, wherein said composition is formulated for mucosal administration.

19. The pharmaceutical composition according to any of claims 15 to 18, wherein said composition is a vaccine.

20. A kit for the immunization of a mammal comprising
- a composition according to claim 15, 17 or 18 when dependent on claim 15; and
- a composition comprising an adjuvant together with a pharmaceutically compatible vehicle,
for simultaneous separate or sequential use in the immunization said mammal.

21. A fusion protein according to any of claims 1 to 6, a chimaeric viral particle according to claim 10 or 11, an extract from a plant according to claim 12 and/or plant cells according to claim 13 or 14, for use as a medicament.

22. Use of the fusion protein according to claim 1, of a chimaeric viral particle according to claim 10 or 11 when dependent on claim 1, of a plant according to claim 12 when dependent on claim 1, or of plant cells according to Claim 13 or 14 when dependent on claim 1, for the preparation of a pharmaceutical composition for the treatment of a pathology associated with a bacterial or viral pathogenic agent.

23. Use of the fusion protein according to any one of the claims from 2 to 6, of a chimaeric viral particle according to claim 10 or 11 when dependent on any of claims 2 to 6, of a plant according to claim 12 when dependent on any of claims 2 to 6, or of cells according to claim 13 or 14 when dependent on any of claims 2 to 6, for the preparation of pharmaceutical compositions for the treatment of a pathology associated with a pathogenic agent selected from the group comprising HIV, HCV, EBV and influenza virus.

24. Use of the fusion protein according to claim 1, of a chimaeric viral particle according to claim 10 or 11 when dependent on claim 1, of a plant according to claim 12 when dependent on claim 1, or of plant cells according to claim 13 or 14 when dependent on claim 1, for deriving diagnostic methods for a pathology associated with a bacterial or viral pathogenic agent.

25. Use of the fusion protein according to any one of the claims from 2 to 6, of a chimaeric viral particle according to claim 10 or 11 when dependent on any of claims 2 to 6, of a plant according to claim 12 when dependent on any of claims from 2 to 6, or of cells according to claim 13 or 14 when dependent on any of claims 2 to 6, for deriving diagnostic methods for a pathology associated with a pathogenic agent selected from the group consisting of HIV, HCV, EBV and influenza virus.

26. Use of an antigenic determinant as defined in the claims 1 to 5 or of an antigen as defined in claim 3, for the preparation of the fusion protein according to any of claims 1 to 6, of the chimaeric viral particle according to claim 10 or 11, plant according to claim 12 or of plant cells according to claim 13 or 14.

27. Use of a capsid protein of PVX for the preparation of the fusion protein according to any of claims 1 to 6, of the chimaeric viral particle according to claim 7, plant according to claim 8 or of cells according to claim 9 or 10.

28. Use of the plant according to claim 12 or of the cells according to claim 13 or 14, for the production of a chimaeric viral particle according to claim 10 or 11.

29. A process for the preparation of a fusion protein according to any of claims 1 to 6 comprising the following steps:
a. cloning the capsid protein as defined in claim 1 or 6, in a vector; and
b. cloning the antigenic determinant as defined in any of claims from 1 to 5 fused to said capsid protein in the vector resulting from step a.

30. A process for the preparation of a chimaeric viral particle according to claim 10 or 11, comprising the step of replacing the capsid protein of a PVX virus, in said PVX virus, with the protein according to any of claims 1 to 6.

31. A process for the preparation of a plant according to claim 12, comprising the following steps:
a1. infecting a plant with a viral particle according to claim 10 or 11;
b1. cultivating the plant obtained as a result of step a1.
c1. extracting said viral particle from said plant.
